# EUROPÄISCHE PATENTSCHRIFT

(11) **EP 0 744 951 B1**
(45) Veröffentlichungstag und Bekanntmachung des Hinweises auf die Patenterteilung: **31.03.2004**
(21) Anmeldenummer: 95906957.6
(22) Anmeldetag: 17.01.1995
(51) Int. Cl.: A61K 31/57, A61K 47/32, A61K 9/06

(54) **VERFAHREN ZUR HERSTELLUNG EINES STERILEN PREDNISOLONGELS**
PROCESS FOR PRODUCING A STERILE PREDNISOLONE GEL
PROCEDE DE PRODUCTION D'UN GEL STERILE DE PREDNISOLONE

(30) Priorität: 17.02.1994 DE 4404990
(43) Veröffentlichungstag der Anmeldung: 04.12.1996
(73) Patentinhaber: Dr. GERHARD MANN chem.-pharm. Fabrik GmbH, D-13581 Berlin (DE)
(72) Erfinder: BELLMANN, Günther, D-13593 Berlin (DE)
(74) Vertreter: Maiwald, Walter, Dr. Dipl.-Chem.
(86) Internationale Anmeldenummer: PCT/EP1995/000155
(87) Internationale Veröffentlichungsnummer: WO 1995/022333

(56) Entgegenhaltungen:
- EP-A- 0 562 445

## Beschreibung

Die Erfindung betrifft ein Verfahren zur Herstellung eines sterilen Prednisolongels.

Prednisolon ist ein Glukocorticoid, dessen enzündungshemmende Wirkung etwa zwei- bis dreimal stärker ist als die des Cortisons. Prednisolon wird als endzündungshemmendes und antirheumatisches Mittel eingesetzt, und zwar bei lokaler Anwendung vorzugsweise auch gegen Dermatosen und Allergien. Als entzündungshemmendes und antiallergisches Mittel wird Prednisolon auch in größerem Umfang in der Augenheilkunde eingesetzt.

Die bisher auf dem Markt erhältlichen Prednisolon-Zubereitungen für die topische Anwendung, insbesondere am Auge, sind entweder wäßrige Suspensionen, da Prednisolon nicht in hinreichender Menge wasserlöslich ist und daher als Suspension eingesetzt werden muß, oder Prednisolonsalben, und zwar meist Suspensionssalben. Der Nachteil bei wäßrigen Suspensionen des Wirkstoffes ist regelmäßig, wie dem pharmazeutischen Technologen bekannt, die Gefahr des sogenannten Caking, d.h. also einer Sedimentbildung, die zum Aufschütteln vor der Applikation nötigt. Dieses Aufschütteln des Medikamentes wird von Laien häufig nicht richtig durchgeführt und hat sowieso den Nachteil, daß damit Dosierungsungenauigkeiten auftreten können. Die Nachteile von Suspensionssalben bestehen in der relativ schlechten Verträglichkeit aufgrund der Teilchengröße und, wie üblich, durch Sehbeeinträchtigungen aufgrund von Schleierbildung, die die Complience bei den Patienten deutlich reduziert. Es besteht daher ein Bedürfnis nach gut verträglichen topischen Anwendungsformen des Prednisolons in Form von Gelen, insbesondere zur Anwendung am Auge. Die Patentanmeldung EP0562445 A2 offenbart ein Gel mit einer Viskosität von 10.000 bis 50.000 mPas, bestehend aus einem Polyacoylat, einem weiteren Polymer oder Polymergemisch, den zur Anwendung am Auge üblichen Hilfsstoffen und gegebenenfalls einem oder mehreren in der Ophthalmologie bekannten Wirkstoffen. Der Herstellung solcher Gele stehen aber beträchtliche Schwierigkeiten entgegen, da wäßrige Suspensionen von Prednisolon oder seiner pharmazeutisch verträglichen Ester nicht durch Autoklavieren sterilisierbar sind, da die Hitzebehandlung die Hydrolyse des Wirkstoffes beschleunigt und ausserdem die Gefahr der Auslösung eines unerwünschten Kristallwachstums besteht. Gele wie beispielsweise Polyacrylatgele sind zwar im Prinzip hitzesterilisierbar, aber bei der Anwendung am Auge sind Isotonisierungsmittel in der Zubereitung erforderlich und diese Isotonisierungsmittel wie Sorbitol, Glycerin u.ä. neigen durch Hitzebehandlung zur Verfärbung oder, mit anderen Worten, durch die Hilfsstoffe erhalten Polyacrylatgele nach dem Autoklavieren eine bräunliche Farbe, die vom Patienten nicht akzeptiert wird.

Die Patentanmeldung WO 86/03966 zeigt die Herstellung eines Fusidinsäure-Augengels auf Basis von Polyacrylaten unter Umgehung der Hitzesterilisation des Wirkstoffs. Hierbei werden zunächst die Hilfsstoffe wie Mannitol, Benzalkoniumchlorid und Tetracemindinatrium in sterilem Wasser gelöst und in dieser Lösung das Carbopol 934 suspendiert. Diese Suspension wird 20 Minuten bei 120°C autoklaviert. Anschliessend wird die sterile mikronisierte Fusidinsäure aseptisch eingearbeitet und die Suspension durch Zugabe von steriler Natronlauge geliert.

Erfindungsgemäß wird jetzt ein Verfahren zur Herstellung eines sterilen Prednisolongels vorgeschlagen, das die Merkmale des Anspruchs 1 anführt.

Es hat sich gezeigt, daß sterile Prednisolongele auf Polyacrylatbasis herstellbar sind, wenn bei der Herstellung bestimmte Verfahrensschritte eingehalten werden. Erfindungsgemäß wird eine wäßrige Polyacrylatsuspension hergestellt, und zwar unter sterilen Bedingungen, und diese dann autoklaviert. Diese Acrylatsuspension wird dann mit einer sterilfiltrierter Lösung von Konservierungsmitteln, Isotonisierungsmitteln und Chelatisierungsmitteln versetzt. Nach sorgfältigem Durchmischen des Ansatzes wird dann durch Zugabe von sterilfiltrierter Natronlauge die Gelbildung eingeleitet und bis zur Homogenität des Gels weitergerührt. Zwischenzeitlich wird das Prednisolon oder seine Ester sterilisiert, und zwar durch Lösen der Substanz in einem geeigneten Lösungsmittel wie beispielsweise Ethylacetat, Sterilfiltrationen dieser Lösung, Fällen des Wirkstoffes beispielsweise durch Zugabe von sterilem Wasser und anschließender Mikronisierung unter aseptischen Bedinungen. Das mikronisierte sterile Prednisolon oder seine Ester werden dann als Gesamtmenge mit etwa der drei- bis zehnfachen Menge Gelgrundlage verrieben. Das restliche Gel wird dann unter gleichmäßiger Durchmischung in das Konzentrat eingerührt. Die fertige Gelzubereitung wird dann in an sich bekannter Weise unter sterilen Bedingungen abgefüllt. In einer alternativen Variante kann bei großem Ansetzen auch das mikronisierte sterile Prednisolon oder seine Ester in einem Teil des wässrigen Isotonisierungsmittels suspendiert werden. Das Polyacrylatgel wird in an sich bekannter Weise bis auf diese Menge des Isotonisierungsmittels fertigge stellt und abschließend wird die Suspension des Prednisolon im Isotonisierungsmittel unter sterilen Bedingungen homogen dem Polyacrylatgel zugemischt.

Diese sterilen Gele werden vom Patienten gut akzeptiert, da ihre Verwendung nicht die Nachteile der fetthaltigen bisher bekannten Salben aufweist. Stabilitätsprüfungen haben ergeben, daß die Gele eine relativ lange Lagerzeit ohne Veränderung der physikalischen Daten, insbesondere ohne Kristallwachstum des Wirkstoffes, aufweisen. Derartige sterile Gelzubereitungen stellen daher insbesondere auf dem Gebiet der Augenheilkunde eine deutlich verbesserte Applikationsform dar.

Die Erfindung wird im folgenden anhand eines Beispieles näher erläutert:

### Beispiel

Wegen der besseren Handhabung wird eine größere Menge Gel als benötigt hergestellt. Das zu verwendende Wasser für Injektionszwecke wird am Tag der Herstellung des Geles produziert.

Zur Herstellung von 500 g Polyacrylatgel werden 1,220 g Polyacrylsäure (erhältlich unter der Bezeichnung "Carbopol 980 NF") mit Hilfe eines Ultraschallgerätes in ca. 700 ml Wasser für Injektionszwecke sorgfältig suspendiert und 20 Minuten bei 121° C und 2 bar autiklaviert. In 700 ml sterilem Wasser für Injektionszwecke werden dann 0,050 g Benzalkoniumchlorid, 20,000 g Sorbitol und 0,050 g des Dinatrium EDTA t x 2H₂O gelöst und über einen Sterilfilter (Sartorius Cellulose-Nitrat-Filter, Bestell-Nr. 11307-50ACN, 0,2 µm) in eine sterile Vorlage filtriert. Die sterilfiltrierte Salzlösung wird unter kräftigem Rühren in die autiklavierte Polyacrylatsuspension gegeben. Der Ansatz wird mit sterilem Wasser auf 1958,121 g aufgefüllt und noch weitere 5 bis 10 Minuten gerührt. Dann werden 0,465 g festes Natriumhydroxid in genau 40 g Wasser für Injektionszwecke gelöst. Die Natronlauge wird tropfenweise unter Rühren über einen Sterilfilter (Millex-GS, 0,22 µm, SLGS 025 BS der Fa. Millipore) zum Ansatz gegeben. Bis zur Bildung eines vollständig homogenen Gels wird gerührt.

5 g steriles, mikronisiertes Prednisolonacetat wird dann mit etwa 30 bis 50 g des Geles langsam und vorsichtig verrührt. Die Herstellung des Prednisolonacetates erfolgt durch Auflösen der Substanz in Ethylacetat, Sterilfiltration der Lösung, Ausfällen mit Wasser und Mikronisierung unter sterilen Bedingungen in an sich bekannter Weise. Nachdem die Substanz gleichmässig in der Gelmenge suspendiert ist, wird der Rest des Geles, also insgesamt 495 g, in 100er Schritten zum Ansatz gegeben uns sorgfältig eingearbeitet. Sämtliche Verfahrensschritte werden unter aseptischen Bedingungen durchgeführt.

Das fertige Gel wird dann ebenfalls unter aseptischen Bedingungen in Tuben abgefüllt. Bei der alternativen Arbeitsweise wird das sterile mikronisierte Prednisolonacetat in der steril filtrietern Isotonisierungslösung aus 700 ml Wasser, 0,050 g Benzalkoniumchlorid, 20,000 g Sorbitol und 0,050 g des Dinatrium EDTA suspendiert und diese Lösung dann, wie bereits beschrieben, unter kräftigem Rühren in die autoklavierte Polyacrylatsuspension eingearbeitet. Die weitere Bearbeitung entspricht der beschriebenen Herstellung des sterilen Polyacrylatgeles.

## Patentansprüche

1. Verfahren zur Herstellung eines sterilen Prednisolongels, **dadurch gekennzeichnet, dass** ein Polyacrylatgel steril dadurch hergestellt wird, dass eine wässrige Polyacrylatsuspension autoklaviert, ggf. mit sterilen Hilfsstoffen versetzt und durch Zugabe von entsprechenden Mengen steriler Natronlauge in das Gel überführt wird und dass Prednisolon oder seine pharmazeutisch annehmbaren Ester in einem geeigneten Lösungsmittel gelöst, steril filtriert, ausgefällt und unter sterilen Bedingungen mikronisiert werden und in entsprechender Menge unter aseptischen Bedingungen in das Polyacrylatgel eingearbeitet werden.

2. Verfahren nach Anspruch 1 **dadurch gekennzeichnet, dass** die sterile Polyacrylatsuspension mit der sterilen wässrigen Lösung eines pharmazeutisch annehmbaren Konservierungsmittels, insbesondere Benzalkoniumchlorid, versetzt wird.

3. Verfahren nach Anspruch 1 und 2 **dadurch gekennzeichnet, dass** die sterile Polyacrylatsuspension mit der sterilen Lösung eines Komplexbildners, insbesondere EDTA und seiner pharmazeutisch annehmbaren Salze, versetzt wird.

4. Verfahren nach Anspruch 1 bis 3, **dadurch gekennzeichnet, dass** die sterile Polyacrylatsuspension mit der sterilen wässrigen Lösung eines Isotonisierungsmittels, insbesondere Sorbitol, versetzt wird.

5. Verfahren nach Anspruch 1 bis 4, **dadurch gekennzeichnet, dass** die gewünschte Gesamtmenge Prednisolon oder seines Esters unter aseptischen Bedingungen mit etwa 1/10 der insgesamt benötigten Polyacrylatgelmenge verrieben und dann in homogener Mischung mit dem restlichen Gel weiterverarbeitet werden.

6. Verfahren zur Herstellung eines sterilen Prednisolongels nach Anspruch 1 bis 4, **dadurch gekennzeichnet, dass** das sterile Prednisolon oder seine Ester mit der sterilen wässrigen Lösung eines Isotonisierungsmittels, ggf. in Kombination mit einem Konservierungs- und Komplexierungsmittel suspendiert und mit der sterilen Polyacrylatsuspension homogen vermischt und diese in ein Gel überführt wird.

## Claims

1. Method of preparation for a sterile prednisolone gel,
**characterized in that** a sterile polyacrylate gel is produced **in that** an aqueous polyacrylate suspension, which comprises optionally sterile excipients, is autoclaved and is transformed into a gel by addition of respective amounts of sterile sodium hydroxide solution and that prednisolone or its pharmaceutically acceptable esters are dissolved in a suitable solvent, sterile filtrated, precipitated and micronized unter sterile conditions and worked into the polyacrylate gel under aseptic conditions.

2. Method according to claim 1,
**characterized in that** an aqueous solution of a pharmaceutically acceptable conserving agent, preferably benzalkonium chloride is added to the sterile polyacrylate suspension.

3. Method according to claims 1 and 2,
**characterized in that** a sterile solution of a complex forming agent, preferably EDTA and its pharmaceutically acceptable salts, is added to the sterile polyacrylate suspension.

4. Method according to any of claims 1 - 3,
**characterized in that** a sterile aqueous solution of an isotonic agent, preferably sorbitol, is added to the sterile polyacrylate suspension.

5. Method according to any of claims 1 - 4,
**characterized in that** the desired total amount of prednisolone or its esters is powdered under aseptic conditions together with approximately 1/10 of the polyacrylate gel amount, which is totally needed, and then is further processed in a homogeneous mixture with the remaining gel.

6. Method for preparation of a sterile prednisolone gel according to any of claims 1 - 4,
**characterized in that** the sterile prednisolone or its esters are suspended with a sterile aqueous solution of an isotonic agent, optionally in combination with a conserving and complex forming agent, and are homogenously mixed with a sterile polyacrylate suspension, this mixture being transformed into a gel.

## Revendications

1. Procédé de production d'un gel stérile de prednisolone, **caractérisé en ce que** l'on prépare un gel de polyacrylate stérile en autoclavant une suspension aqueuse de polyacrylate, en l'additionnant éventuellement d'adjuvants stériles puis en la transformant en gel par addition de quantités appropriées d'hydroxyde de sodium stérile, et **en ce que** la prednisolone ou son ester pharmaceutiquement acceptable est dissous(te) dans un solvant approprié, stérilisé(e) par filtration, précipité(e) et micronisé(e) dans des conditions stériles avant d'être incorporé(e) en quantité appropriée et en conditions aseptiques dans le gel de polyacrylate.

2. Procédé selon la revendication 1, **caractérisé en ce que** la suspension stérile de polyacrylate est mélangée à la solution aqueuse stérile d'un conservateur pharmaceutiquement acceptable, en particulier le chlorure de benzalkonium.

3. Procédé selon les revendications 1 et 2, **caractérisé en ce que** la suspension stérile de polyacrylate est mélangée à la solution stérile d'un agent complexant, en particulier l'EDTA (acide éthylènediamminetétraacétique) et ses sels pharmaceutiquement acceptables.

4. Procédé selon les revendications 1 à 3, **caractérisé en ce que** la suspension stérile de polyacrylate est mélangée à la solution aqueuse stérile d'un isotonisant, en particulier le sorbitol.

5. Procédé selon les revendications 1 à 4, **caractérisé en ce que** la quantité totale souhaitée de prednisolone ou de son ester est triturée en conditions aseptiques avec environ 1/10 de la quantité de gel de polyacrylate requise au total, puis incorporée en mélange homogène avec le reste du gel.

6. Procédé de fabrication d'un gel de prednisolone stérile selon les revendications 1 à 4, **caractérisé en ce que** la prednisolone stérile ou son ester est mis(e) en suspension dans la solution aqueuse stérile d'un isotonisant, éventuellement en combinaison avec un conservateur et un agent complexant, et est mélangé(e) de manière homogène à la suspension stérile de polyacrylate, et **en ce que** cette dernière est transformée en un gel.
